# EUROPEAN PATENT APPLICATION

(11) **EP 0 559 569 A1**
(43) Date of publication of application: **08.09.1993**
(21) Application number: 93400565.3
(22) Date of filing: 04.03.1993
(51) Int. Cl.: C07C 323/25, C07C 323/14, C07C 323/41, C07C 323/42, C07C 31/28, A61K 31/135, A61K 31/165

(54) **Central nervous system antiischemic agents**

(30) Priority: 06.03.1992 US 847389
(71) Applicant: Bristol-Myers Squibb Company, New York, N.Y. 10154 (US)
(72) Inventor: Poindexter, Graham S., Old Saybrook, Connecticut 06475 (US)
(74) Representative: Durand, Yves Armand Louis

(57) **Abstract**

A series of phenylalkylaminoalkyl derivatives of Formula I 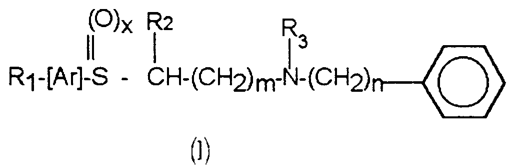
wherein Ar is naphtyl or phenyl;
R¹ is hydrogen, fluoro or R⁴CONH-;
R² is hydrogen or C₁₋₆ alkyl;
R₃ is C₁₋₆ alkyl;
R⁴ is C₁₋₆ alkyl or phenyl- C₁₋₆ alkyl;
x is zero or the integers 1 and 2;
m is selected from the integers 1 to 6; and
n is selected from the integers 2 and 3,
has been found to provide effective antiischemic protection for CNS tissue, particularly neurons. A method of treatment to protect against CNS ischemia, such as that resulting from trauma or stroke or other ischemic conditions, comprises administration of these novel compounds to an individual in need of such treatment.

## Description

### Background of The Invention

This invention pertains to arylsulfur carbon compounds having drug and bio-affecting properties and to their preparation and use. In particular the invention is concerned with phenylalkylaminoalkyl derivatives of arylsulfide, sulfinyl and sulfonyl compounds.

Related art in terms of chemical structure may be viewed in terms of the following general structure (1). 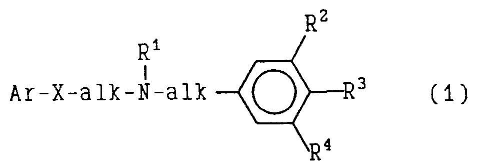
in which Ar is an optimally substituted phenyl group;
X can be S, SO or SO₂; R¹ is hydrogen or alkyl; alk is alkyl; and R², R³, and R⁴ are various substituents but not all are hydrogen simultaneously (i.e., not an unsubstituted phenyl ring).

In UK Patent Application GB 2,088,873A, a series of N-arylalkyl phenylalkylamine derivatives (2) with positive inotropic activity and negative chronotropic action was disclosed for use in cardiovascular diseases such as coronary heart disease and cardiac arrhythmias. 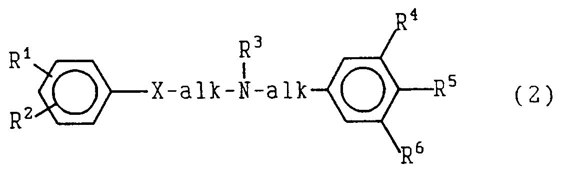
In formula (2), X is O, S, SO or SO₂; and 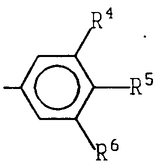
is at least a di-substituted phenyl moiety.

US 4,959,366 discloses antiarrhythmic agents of formula (3). 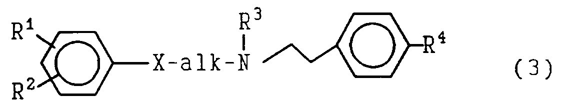
In formula (3), X is O, S or a chemical bond and 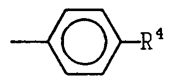
is a substituted phenyl ring. The art compounds of formulas (2) and (3) are recognized as being useful due to their positive inotropism and negative effects on cardiac frequency.

By contrast the compounds of the present invention (3) are not positive inotropes 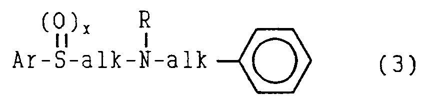
but have properties that render them useful medicaments for treating ischemic and traumatic injuries to the central nervous system. As such, the instant compounds will be applicable for treatment of conditions such as post-traumatic injury of the head and spinal cord, focal ischemia, stroke (a focal ischemia with reperfusion), global ischemia (resulting from cardiac arrest, suffocation, etc.), hemorrhagic and radiation injuries.

CNS tissue, especially brain cells, are particularly vulnerable to damage caused by ischemic conditions. Brain ischemia, or insufficient oxygen, may result from injury or disease and may last from only transient periods of time to periods of lengthy duration, as in stroke or following cardiac arrest. The principle disease associated with brain ischemia is ischemic cerebral vascular disease (ICVD), and in general refers to the disorders resulting from an insufficient supply of blood, with its nourishing oxygen content, to brain areas.

Seizures and stroke are two frequent manifestations of ischemic cerebral vascular disease. There can be many underlying causes of cerebral vascular ischemia, with several of the more common listed below:
- Atherosclerosis -: the most common underlying condition and may be caused either by hemodynamic or thromboembolic mechanisms.
- Hypertension -: can impair cardiac function and/or alter cerebral circulation.
- Cardiac disease -: comprises such disorders as cardiac arrhythmias, congestive heart failure, myocardial infarction, and the like.
- Hematologic disorders -: increased viscosity of blood or other deleterious blood alterations.
- Arteritis -: inflammation of intracranial arteries, may be of either infective or noninfective origin.
- Head injury -: injuries compromising cerebral blood supply or altering the cerebral circulation.
- Vasospasm -: decreased blood flow due to constrictive spasms of the vasculature.

As a result of ischemia in the cerebral vasculature, various clinical disorders may result. These disorders may range from transient ischemic attacks to a permanently disabling stroke. Transient ischemic attacks (TIAs) are brief spells (usually less than one hour) characterized by symptoms referable to vascular territories in the retinal, carotid, or vertibrobasilar circulation. These spells indicate a high risk for subsequent stroke, a serious disorder of more lengthy duration. Stroke, also called "cerebral vascular accident" or "cerebral crisis" refers to a sudden apoplectic attack ordinarily due to cerebral infarction, hemorrhage, or vasospasm and usually characterized by some degree of paralysis and/or deficits in speech or memory. The immediate 72 hours following stroke onset is a critical time interval since the degree of brain damage suffered may be reversible to some extent if the anoxic conditions can be eased. When the brain is deprived of oxygen, due to stroke, heart attack, or other cause, nerve damage can become irreversible and virtually untreatable. It is one object of this invention to prevent the nerve cell damage caused by ischemia. Current methods for treating stroke and brain ischemia necessarily focus on preventing hypoxia and restoring adequate blood circulation. There are no medically accepted methods for protecting brain cells subjected to ischemic conditions.

Various therapies have been employed in efforts to treat acute strokes and prevent significant persisting neurological deficits. Types of agents employed have been vasodilators, such as papaverine, prostacyclin, and pentoxifylline: hemodilution agents, thrombolytic agents such as tissue plasminogen activator (TPA) and calcium channel blockers: and anticoagulants such as heparin or warfarin. Transient ischemic attacks are generally treated with either anticoagulants or antiaggregators such as aspirin.

There are, at present, no effective, approved treatments which may be used therapeutically and/or prophylactically to minimize the brain cell damage caused by ischemic conditions. It is another objective of this invention to provide such a treatment that will protect brain cells from ischemia-induced damage.

Several compounds have been disclosed as being under study as potential protective agents for anoxic brain cells.

An experimental drug, MK-801, is being studied to determine its effectiveness in limiting neuronal injury after ischemia (Barnes, Science, Vol. 235, pages 632-633, February 6, 1987).

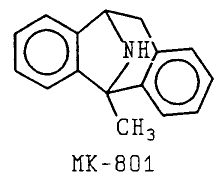

Dextromethorphan, the non-narcotic cough suppressant, and ketamine have also been disclosed as being effective in protecting brain cells under anoxic conditions by Simon in "Frontiers in Excitatory Amino Acid Research", pages 639-644, 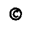 1988 Alan R. Liss, Inc.

Gotti, et al., JPET. 247/3, pages 1211-1221 (1988): have disclosed that ifenprodil and an analog are effective in tissue sparing in animal models of stroke and brain infarction.

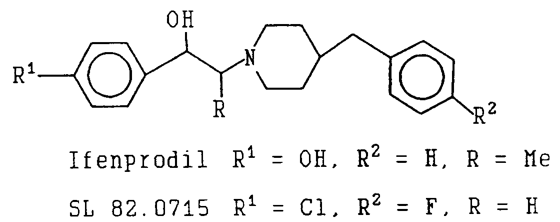

A series of anti-anoxic 2-[4-benzoyl-1-piperidinyl)-1-phenylalkanol derivatives, having some structural resemblance to ifenprodil type compounds, is disclosed in U.S. Pat. No. 4,711,899 issued in December, 1987 to Gaudilliere, et al.

Wauquier, et al., in "Drug Development Research", 8/373-380 (1986) disclosed that Sabeluzole (R 58,735) is a potent antihypoxic agent with anticonvulsant properties.

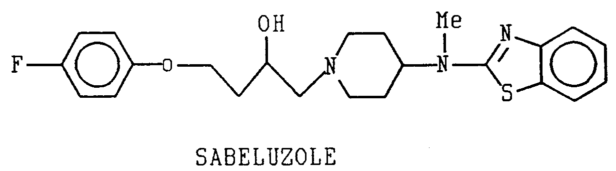

An antiischemic agent, E-2001, was disclosed by Kaneka, et al., 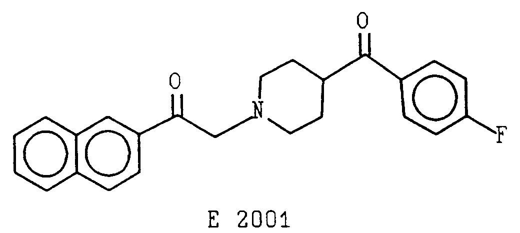
Arzneim.-Forsch./Drug Res., 1989, 39, 445; as showing neuronal protection in a gerbil model of forebrain ischemia.

Emopamil, a calcium and serotonin antagonist, has been reported to provide protective effects in focal 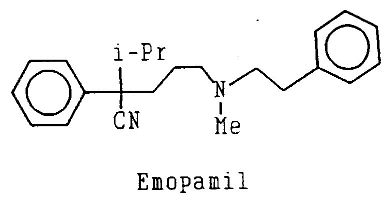
stroke models as well as in models of gerbil forebrain ischemia; see, e.g. Hoffman, et al., Arzneim.-Forsch./Drug Research, 1989, 39, 304; Szabo, ibid., 1989, 39, 309.

Dextraze, et al., claimed a series of 5-halo-pyrimidin-2-ylpiperazinylalkyl derivatives containing, inter alia, compounds of formula (4) all of which are 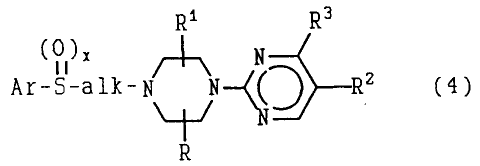
disclosed as having useful anti-ischemic properties for treatment and prevention of disorders resulting from brain and/or spinal cord anoxia.

There is nothing in any of the foregoing references, or in the general prior art, to suggest the instant novel antiischemic compounds and their application to anoxia-related CNS disorders.

### Summary of The Invention

The present invention is concerned with certain phenylalkylaminoalkyl derivatives of some arylsulfur compounds and with a method of treatment employing these novel agents. The method is intended for primarily protecting CNS tissue, particularly neurons against the effects of ischemia which can result from trauma or disorders such as stroke. The method essentially involves administration of novel compounds of this invention to a mammal in need of such treatment.

### Detailed description of the Invention

In its broadest aspect, the present invention comprises phenylalkylaminoalkyl derivatives of arylsulfur compounds having anti-ischemic properties and characterized by Formula I 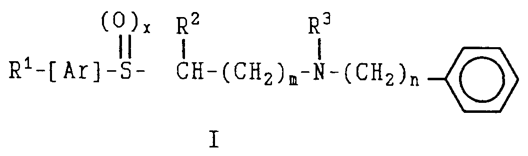
wherein Ar is naphthyl or phenyl, unsubstituted or monosubstituted with fluoro or R⁴CONH- as R¹ is hydrogen, fluoro or R⁴CONH; R² is hydrogen or lower alkyl; R³ is lower alkyl; R⁴ is lower alkyl or phenyl-lower alkyl; x is zero and the integer 1 and 2; m is selected from the integers 1, 2, 3, 4 or 5; and n is independently selected from the integers 2 and 3.

Lower alkyl denotes alkyl groups containing from one to six carbon atoms (C₁₋₆). Pharmaceutically acceptable salts and/or solvates of the Formula I compounds also comprise the present invention which further includes stereoisomers, such as enantiomers, which can arise as a consequence of structural asymmetry in selected Formula I compounds. Separation of individual isomers is accomplished by application of various methods and procedures well known to practitioners' in the art.

Preferred compounds of Formula I comprise structures wherein R¹ is 4-fluoro, R³ is methyl, x is zero, m is 2 or 3, and n is 2. The most preferred compound is N-[3-[(4-fluorophenyl)thio]propyl]-N-methyl-benzene-ethanamine.

For medicinal use, the pharmaceutically acceptable acid addition salts, those salts in which the anion does not contribute significantly to toxicity or pharmacological activity of the organic cation, are preferred. The acid addition salts are obtained either by reaction of an organic base of structure I with an organic or inorganic acid, preferably by contact in solution, or by any of the standard methods detailed in the literature available to any practitioner skilled in the art. Examples of useful organic acids are carboxylic acids such as maleic acid, acetic acid, tartaric acid, propionic acid, fumaric acid, isethionic acid, succinic acid, pamoic acid, cyclamic acid, pivalic acid and the like: useful inorganic acids are hydrohalide acids such as HCl, HBr, HI; sulfuric acid: phosphoric acid and the like. The preferred solvate forms of Formula I compounds are hydrates.

The compounds of the present invention are useful pharmacologic agents with anti-ischemic properties. Central nervous system tissue is particularly vulnerable to damage caused by ischemic conditions. Brain ischemia, or insufficient oxygen, may result from injury or disease and may last from only transient periods of time to periods of lengthy duration, as in stroke. In this regard, the compounds of Formula I are useful for treatment and prevention of injury to the brain and spinal cord and of edema due to head trauma, stroke, arrested breathing, cardiac arrest, Rey's syndrome, cerebral thrombosis, embolism, hemorrhage or tumors, encephalomyelitis, spinal cord injury, hydroencephalis, and post-operative brain injury.

The anti-ischemic activity of the compounds of Formula I have been demonstrated in certain pharmacologic tests and model systems that are used to determine drug effects on CNS tissue, particularly brain ischemia and its aftermath.

From results of in vitro tests with vascular and neuronal preparations, the Formula I compounds demonstrate serotonin type 2 (5HT2) and calcium antagonism as well as partial agonism at the 5HT-1A receptor site. Importantly, in vascular smooth muscle preparations, they exhibit greater potency centrally than peripherally. Competitive antagonism at the polyamine binding site associated with the N-methyl-d-aspartate (NMDA) glutamate receptor has also been observed in testing representative members of the series. In studies of an anoxic depolarization response to hypoxic conditions measured in vitro, these compounds diminish the amplitude and increase the latency period.

More significant however are results seen with in vivo testing. Compounds of Formula I promote survival under anoxic (100% nitrogen) conditions. Specifically, administration of the compounds of Formula I results in protecting against hypoxia-induced death in an anoxic nitrogen test in rats. This particular test identifies the neuro-protective effects of substances against lethality produced by a lack of oxygen consumption (anoxia). In this test procedure, control animals exposed for one minute to a pure nitrogen atmosphere will expire because of respiratory failure attributed to irreversible damage to the brain respiratory center. The animals exhibit strong heartbeat following anoxia exposure. To demonstrate effectiveness, experimental compounds must antagonize the anoxic insult resulting in survivability of the test animals.

Most relevant however is testing against true cerebral ischemia in vivo. Two test model systems have been used:
1. transient global forebrain ischemia (in the Mongolian gerbil);
2. permanent focal ischemia (in the spontaneously hypertensive rat).

In both of these paradigms, cerebral tissue damage is reduced with Formula I compound treatment. In cases of the most preferred compounds, significant reduction of cerebral tissue damage is seen even when drug administration is delayed for an hour following vessel occlusion.

One aspect then of the present invention involves administration of a compound of Formula I or a pharmaceutically acceptable acid and/or solvate thereof, to a mammal suffering from ischemia or being susceptible to ischemia. In general the compound would be given in a dose range of from about 1.0 mg/kg to about 50 mg/kg body weight.

Although the dosage and dosage regimen of a Formula I compound must in each case be carefully adjusted, utilizing sound professional judgment and considering the age, weight and condition of the recipient, the route of administration and the nature and extent of the ischemia, generally, the daily dose for human use will be from about 0.5 g to about 10 g, preferably 1 to 5 g. In some instances, a sufficient therapeutic effect can be obtained at lower doses while in others, larger doses will be required. As is apparent to one skilled in clinical pharmacology, the amount of a Formula I compound comprising the daily dose may be given in a single or divided dose, taking into account those principles understood by the skilled practitioner and necessary for his practice of the art.

The term "systemic administration" as used herein refers to oral, sublingual, buccal, transnasal, transdermal, rectal, intramuscular, intravenous, intraventricular, intrathecal, and subcutaneous routes. In accordance with good clinical practice, it is preferred to administer the instant compounds at a concentration level which will produce effective beneficial effects without causing any harmful or untoward side effects.

Therapeutically, the instant compounds are generally given as pharmaceutical compositions comprised of an effective ischemia-protective amount of a Formula I compound or a pharmaceutically acceptable acid addition salt and/or hydrate thereof and a pharmaceutically acceptable carrier. Pharmaceutical compositions for effecting such treatment will contain a major or minor amount (e.g. from 95% to 0.5%) of at least one compound of the present invention in combination with a pharmaceutical carrier, the carrier comprising one or more solid, semi-solid, or liquid diluent, filler and formulation adjuvant which is non-toxic, inert and pharmaceutically acceptable. Such pharmaceutical compositions are preferably in dosage unit forms; i.e., physically discrete units having a predetermined amount of the drug corresponding to a fraction or multiple of the dose which is calculated to produce the desired therapeutic response. In usual practice, the dosage units contain 1, 1/2, 1/3, or less of a single dose. A single dose preferably contains an amount sufficient to produce the desired therapeutic effect upon administration at one application of one or more dosage units according to the predetermined dosage regimen, usually a whole, half, third, or less of the daily dosage administered once, twice, three, or more times a day. It is envisioned that other therapeutic agents can also be present in such a composition. Pharmaceutical compositions which provide from 0.1 to 1 g of the active ingredient per unit dose are preferred and are conventionally prepared as tablets, lozenges, capsules, powders, aqueous or oily suspensions, syrups, elixirs, and aqueous solutions. Preferred oral compositions are in the form of tablets, capsules, and may contain conventional excipients such as binding agents (e.g., syrup, acacia, gelatin, sorbitol, tragacanth, or polyvinylpyrrolidone), fillers (e.g. lactose, sugar, maize-starch, calcium phosphate, sorbitol or glycine), lubricants (e.g. magnesium stearate, talc, polyethylene glycol or silica), disintegrants (e.g. starch) and wetting agents (e.g. sodium lauryl sulfate). Solutions or suspensions of a Formula I compound with conventional pharmaceutical vehicles are employed for parenteral compositions such as an aqueous solution for intravenous injection or an oily suspension for intramuscular injection. Such compositions having the desired clarity, stability and adaptability for parenteral use are obtained by dissolving from about 0.1% to 10% by weight of a Formula I compound or one of its salt forms in water or a vehicle consisting of a polyhydric aliphatic alcohol such as glycerine, propylene glycol, and the polyethylene glycols or mixtures thereof. The polyethylene glycols consist of a mixture of non-volatile, usually liquid, polyethylene glycols which are soluble in both water and organic liquids and which have molecular weights from about 200 to 1500.

When transnasal application is intended, the Formula I compound pharmaceutical composition is formulated in a pharmaceutical composition which enhances penetration of the nasal mucosa. Such formulations normally employ fatty acid salts of the Formula I base compound and their preparation and use would be known to one skilled in the pharmaceutical arts.

The general procedures for preparation of Formula I compounds are outlined in Schemes 1 and 2. In these schemes, the formulas Ia, Ib, and Ic refer to structural subclasses of the sulfur moiety as Ia are sulfides (x = 0); Ib are sulfinyl compounds (x = 1); and Ic are sulfonyl compounds (x = 2). In both schemes Q is an organic synthetic leaving group, preferably chloro or bromo. Organic leaving groups and their manipulations are well known to one skilled in organic synthesis.

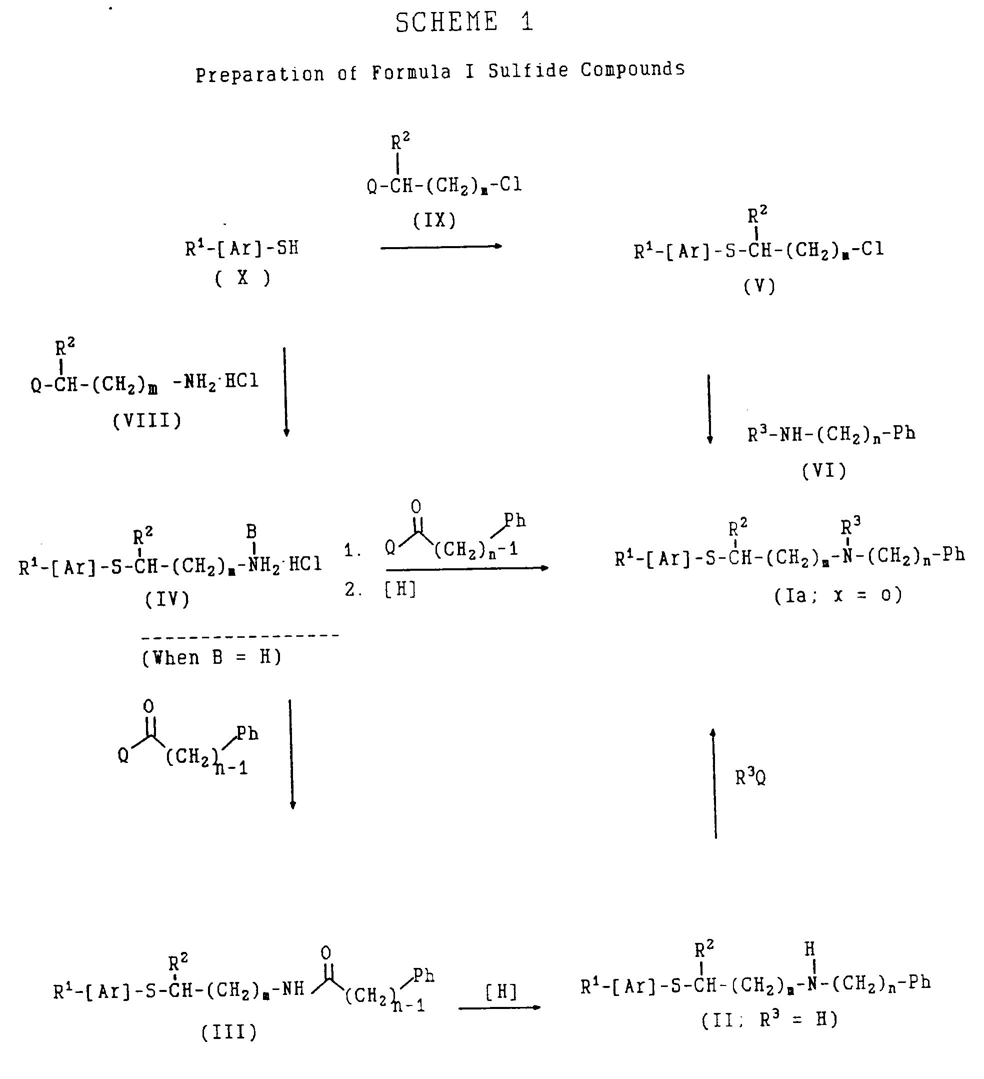

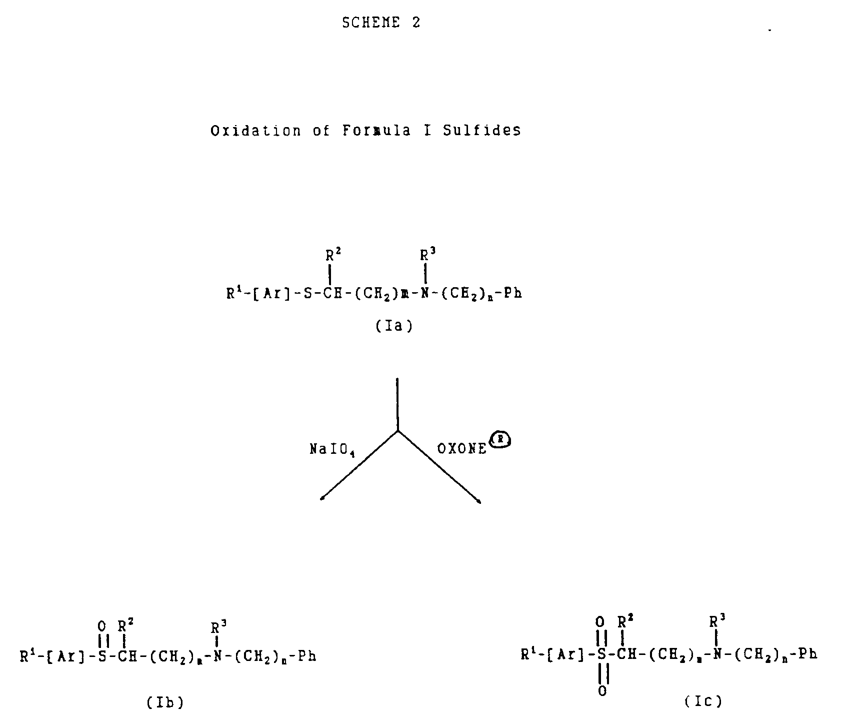

Scheme 1 sets out two synthetic routes which, diverging from the starting appropriate aryl thiol compound (X), then converge at the aminoalkyl sulfide product, Ia. In the clockwise loop, the appropriate aryl thiol compound (X) is treated with a strong base such as ethanolic sodium ethoxide and the resulting thiol anion is then alkylated with a selected dihaloalkane such as dibromoethane, 1-bromo-3-chloropropane, 1-bromo-4-chlorobutane, and the like to furnish compound V, an intermediate haloalkyl sulfide compound in high to quantitative yields. These formula V intermediates were generally allowed to react without further purification with appropriate amines VI to afford the respective aminoalkyl sulfide products of Formula Ia. These sulfides were purified by column chromatography and then converted to crystalline salt forms (hydrochloride, maleate, fumarate, oxalate, etc.) to provide water solubility.

The alternate counterclockwise loop of Scheme 1 involves alkylation of compound X with a haloalkylammonium salt (B is H or R³) to yield the aminoalkyl sulfide compound IV which is either reductively acylated to provide the desired Ia product when B is R³ or acylated to give the amide intermediate III when B is hydrogen. The amide III is then reduced, the use of borane dimethyl sulfide complex in tetrahydrofuran being a preferred reduction method, to provide the secondary aminoalkyl sulfide II. This Formula II compound is N-alkylated by alkylation or reductive acylation to yield the desired Ia product. The monomethylation of II, Ia compounds with R³ being methyl are preferred compounds, is conveniently accomplished by treatment of II with ethyl formate followed by reduction of the resulting formamide using the borane dimethyl sulfide method already mentioned.

Scheme 2 displays the oxidative routes employed to prepare the corresponding sulfoxides Ib and sulfones Ic from a selected sulfide Ia. The use of sodium periodate as the oxidant gave the sulfoxides while use of OXONE®, (potassium peroxymonosulfate: 2KHSO₅·KHSO₄·K₂SO₄) afforded the fully oxidized sulfones. The sulfoxides are produced as a racemic mixture.

Amide derivatives (Formula I, R¹ = alkylcarbonylamino) are obtained from amine compounds (Formula I, R¹ = amino) using standard acylation methodology. 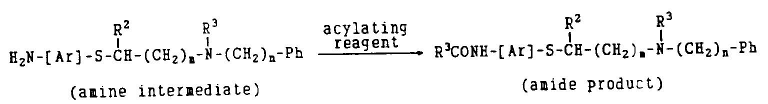
Generally the amide products are easily accessible by using acylating reagents such as excess alkanoic acid anhydrides, acid halides, chloroformate or isocyanate in the presence of an acid scavenger such as excess triethylamine. The formation of bis-acylated adducts was not a problem as their formation did not occur to any appreciable extent.

A limited series of a-substituted sulfones may also be prepared by metalation of a sulfone wherein R² is hydrogen, followed by addition of an electrophilic reagent, such as an alkyl halide, to give e.g. a Ib product where R² is alkyl.

### Description of Specific Embodiments

The compounds which constitute this invention and their methods of preparation will appear more full from a consideration of the following examples which are given for the purpose of illustration only and are not to be construed as limiting the invention in sphere or scope. All temperatures are understood to be in °C when not specified.

The nuclear magnetic resonance (NMR) spectral characteristics refer to chemical shifts (δ) expressed as parts per million (ppm) versus tetramethylsilane (TMS) as reference standard. The relative area reported for the various shifts in the proton NMR spectral data corresponds to the number of hydrogen atoms of a particular functional type in the molecule. The nature of the shifts as to multiplicity is reported as broad singlet (bs), singlet (s), multiplet (m), doublet (d), doublet of doublets (dd), quartet (q) or pentuplet (p). Abbreviations employed are DMSO-d₆ (deuterodi-methylsulfoxide). CDCl₃ (deuterochloroform), and are otherwise conventional. The elemental analyses are reported as percent by weight.

### Synthesis of Intermediates

Aryl thio compounds (X) as well as other conventional intermediates, e.g. VI, VIII, and IX; used in the preparation of aminoalkyl sulfide products Ia were generally commercially available. Representative syntheses of some of these compounds are provided hereinbelow nevertheless.

### Example 1

### 1-Napthalenethiol (X))

Was prepared from 1-naphthanol using the general method of Newman (J. Org. Chem., 1966, 31, 3980). N,N-dimethylthiocarbamyl chloride (49.6 g, 0.400 mole) was slowly added to a solution of 1-naphthol (48.72 g, 0.338 mole), triethylamine (50 g, 0.50 mole) and 200 mg of 4-dimethylaminopyridine (DMAP) dissolved in 200 mL of methylene chloride. The resulting mixture was allowed to reflux for several hours. When all the starting material was consumed, 100 mL of water was added and the phases separated. The organic layer was washed several times with water and brine. After drying (magnesium sulphate), the filtrate was concentrated and the resulting crude material was purified by flash chromatography (SiO₂: EtOAc/Hexane) to give the 1-napthylthiocarbamate as a white solid (33.28 g, 43% yield): mp 116-118°C; ¹H NMR (CDCl₃) δ 7.85 (m, 3H), 7.49 (m, 3H), 7.22 (m, 1H), 3.52 (s, 1H), 3.51 (s, 1H), and 1.55, (s, 1H).

The naphthylthiocarbamate (33.28 g, 0.14 mole) was heated in a sand bath at 279°C for 3 h until all the starting material was consumed. The reaction mixture was then purified by flash chromatography to give 8.7 g (26% yield) of the product as a thick brown oil: ¹H NMR (CDCl₃) δ 8.33 (d, 1H, J = 9 Hz), 7.94 (d, 1H, J = 8.1 Hz), 7.88 (d, 1H, J = 9.0 Hz), 7.78 (d, 1H, J = 7.2 Hz), 7.53 (m, 3H), 3.23 (br s, 1H), 3.02 (br s, 1H), 1.55 (s, 1H).

The carbamate was then cleaved by refluxing the product in 50 mL of aq. NaOH (10% wt/volume) and 50 mL of water. After 2 h the reaction was complete and the solvent was removed in vacuo to yield the crude sodium salt 1-naphthylthiol (3a). This material was then used without further purification in the synthesis of Formula I products.

### Example 2

### N-Methylbenzenepropanamine (VI).

45.0 g of hydrocinnamic acid (0.3 mol) was dissolved in 300 mL anhydrous dichloromethane and treated with some drops of dimethylformamide, followed by 30 mL of thionylchloride (dropwise addition). The solution was stirred at room temperature over night, and solvent and excess thionylchloride were distilled off under reduced pressure (100 mmHg). The residue was taken up twice in dichloromethane, and solvent was removed on the evaporator. The residue was dissolved in 400 mL dichloromethane, cooled with an ice bath, and methylamine gas was bubbled through the stirred and cooled mixture until the pH became basic. The product was filtered off and washed with water/dichloromethane, resulting in 48.9 g of colorless crystals (quantitative). The NMR spectrum indicated high purity and correct structure. 48.8 g of the amide (0.3 mol) was dissolved in hot anhydrous tetrahydrofuran (600 mL) under nitrogen. Borane dimethylsulfide (10 M, 0.9 mol) was added by syringe, and 3 portions of dimethyl sulfide were distilled off. The remaining solution was refluxed for 3 h, at which time some starting material was still present by TLC. Reflux was continued for 1 1/2 h, the mixture was cooled, and the excess of borane was destroyed by slow addition of 6N HCl. The acidic solution was refluxed for 1 h, and concentrated on the evaporator. The residue was extracted with dichloromethane and aqueous sodium hydroxide, giving 50.0 g yellow oil. Distillation at 100 mmHg gave 20.81 of pure oily product, with appropriate NMR, and 7.43 g of somewhat contaminated product (63% yield).

### Example 3

### 1-Chloro-3-[(4-fluorophenyl)thio]propane(V)

To a stirred, room temperature solution of 2.3 g (100 mmol) of sodium metal dissolved in 100 mL of abs ethanol, was added 10.0 g (78.0 mmol) of 4-fluorothiophenol. After 15 min. 15.7 g (100 mmol) of 3-bromo-1-chloropropane was added and the reaction stirred for 17 h at room temperature. The resulting suspension was concentrated in vacuo and the residue taken up in methylene chloride and washed with water and brine. After drying over magnesium sulfate and filtration, the volatiles were removed in vacuo to furnish 15.1 g (73.7 mmol, 94% yield) of the sulfide product as a clear oil: ¹H-NMR (CDCl₃) δ 7.36 (m, 2H), 6.99 (m, 2H), 3.65 (t, 2H, J = 7 Hz), 3.01 (t, 2H, J = 7 Hz), and 2.01 (m, 2H).

### Example 4

### 3-[(4-Fluorophenyl)thio]-N-methyl-1-propanamine hydrochloride (IV).

Solutions of the chloropropane intermediate of Example 3 (20.0 g, 97.8 mmol) in ethanol (10 mL) and methylamine (41 g) in ethanol were mixed at 0°C. The mixture was heated to 60°C for 24 h in a stainless steel bomb. Solvent was evaporated, and the product was extracted with ether/hydrochloric acid. The aqueous phase was made alkaline and the product was extracted with chloroform, yielding 18.51 g of deep yellow oil. Column chromatography yielded 16.45 g of pure amine (85%). The hydrochloride salt was obtained as a colorless solid: mp 117-118°C; ¹H NMR (DMSO-d₆) δ 8.97 (s, 2H), 7.45-7.38 (m, 2H), 7.18 (t, 2H, J = 7 Hz), 3.01 (t, 2H, J = 7 Hz), 2.93 (t, 2H, J = 7 Hz), 2.49 (s, 3H), 1.89-1.79 (q, 2H, J = 7 Hz).

Anal. Calcd. for C₁₀H₁₄FNS·HCl: C, 50.95; H, 6.41; N, 5.94. Found: C, 50.72; H, 6.37; N, 5.98.

### Example 5

### 4-Fluoro-N-methylbenzeneethanamine hydrochloride (IV).

To a mixture of 4-fluorophenylacetic acid (30.8 g, 0.2 mol) and carbonyldiimidazole (32.8 g, 0.20 mmol) under nitrogen was added anhydrous dichloromethane (100 mL). The solution was heated briefly to reflux, and stirred at room temperature for 1 h. Methylamine was bubbled through the solution at room temperature, under stirring. After stirring overnight, the residue was extracted with ethyl acetate/water, giving 34.92 g of crystalline amide (theory 33.4 g). This material was dissolved in hot anhydrous tetrahydrofuran (300 mL) under nitrogen. Borane dimethyl sulfide (10 M, 0.6 mol) was added dropwise, under stirring. The mixture was refluxed for 6 h, cooled and excess borane was destroyed by slow addition of 6 N hydrochloric acid. The acidic mixture was stirred at room temperature over night, solvents were partially removed on the evaporator, and the product was extracted with ether/acidic water. The aqueous phase was made alkaline and the product was extracted with dichloromethane, giving 32 g of crude oil with a complex NMR spectrum. Distillation gave 12.02 g of colorless, oily product, with acceptable NMR indicating this to be a useful intermediate.

### Example 6

### N-[3-[(4-Chlorophenyl)thio]propyl]amine hydrochloride (IV)

To a stirred, room temperature, N₂-atmosphere solution of freshly prepared sodium ethoxide (70 g, 1.5 mole) in 600 mL of ethanol, was added 4-chlorothiophenol (99.5 g, 0.686 mole). After stirring the solution for 10 min, 3-chloropropylamine hydrochloride (101 g, 0.777 mole) was added portionwise. The resulting suspension was stirred 70 h at room temperature. The reaction was then concentrated in vacuo and 500 mL of water added. The aqueous mixture was extracted with ether. The combined organic layers were washed with H₂O, brine, and then dried over potassium carbonate and filtered. After concentration in vacuo, 136 g (0.673 mole, 98% yield) of the product was obtained as a pale yellow oil. A small portion of the oil was converted to the hydrochloride salt: mp 149-165°C (sintered); ¹H NMR (DMSO-d₆) δ 8.27 (br s, 3H), 7.35 (s, 4H), 3.09 (t, 2H, J = 7.1 Hz), 2.86 (t, 2H, J = 7.1 Hz), and 1.86 (p, 2H, J = 7.1 Hz).

Anal. Calcd. for C₉H₁₂ CINS · HCl: C, 45.39; H, 5.51; N, 5.89. Found: C, 45.37; H, 5.55; N, 5.88.

### Example 7

### N-[3-[(4-Chlorophenyl)thio]propyl]benzeneacetamide (III)

A solution of the product of Example 6 (51.9 g, 0.257 mole) was taken up in 300 mL of THF and triethylamine (29.2 g, 0.289 mole) and 4,4-dimethylaminopyridine (DMAP, 10mg) added. After cooling this solution in an ice-bath, a solution of phenylacetyl chloride (42.6 g, 0.275 mole) was slowly added dropwise over a period of 30 min. The resulting suspension was allowed to warm to room temperature and stir 2 h. Water was added and the layers were separated. After the aqueous layer was extracted with ether, the combined organic portions were washed with H₂O and brine, and then dried over anhydrous magnesium sulfate. Filtration and concentration in vacuo furnished 83.5 g of the product as a yellow oil. The crude product was taken up in ethyl acetate and allowed to crystallize overnight in a refrigerator (6°C). In this manner 47.8 g (0.149 mole, 58% yield) of the product was collected by filtration and isolated as a colorless white solid: mp 83-85°C; ¹H NMR (CDCl₃) δ 7.25 (m, 9H), 5.78 (br s, 1H), 3.52 (s, 2H), 3.27 (m, 2H), 2.79 (t, 2H, J = 7.1 Hz), and 1.72 (p, 2H, J = 7.1 Hz).

Anal. Calcd. for C₁₇H₁₈ ClNOS: C, 63.84; H, 5.68; N, 4.38. Found: C, 63.83; H, 5.55; N, 4.34.

### Example 8

### N-[3-[(4-Chlorophenyl)thio]propyl]benzeneethanamine hydrochloride (II)

To a solution of the product of Example 7 (71.2 g, 0.223 mole) in 300 mL of THF under N₂ was slowly added 112 mL of borane-methyl sulfide complex (2.0 M in THF). During this addition phase vigorous gas evolution took place. The resulting solution was stirred at room temperature for 16 h and then refluxed for an additional 3 h, and then cooled to room temperature. Very carefully, 100 mL of 10% aqueous hydrochloric acid (vol:vol) was added and the reaction then refluxed 1 . After cooling to ambient temperature, the reaction mixture was made basic by the addition of 10% aqueous sodium hydroxide (wt:vol). The phases were separated and the organic layer washed with water and brine. After drying over anhydrous magnesium sulfate and filtration, the volatile were removed in vacuo to give 60.1 g of a pale yellow liquid. This material was purified by flash chromatography (SiO₂: acetone/hexane) to furnish 50.4 g (0.165 mole, 74% yield) of product as a pale yellow oil. A small sample of this base was converted to the hydrochloride salt and isolated as a white crystalline solid after recrystallization from ethanol: mp 178-179°C; ¹H NMR (DMSO-d₆) δ 9.30 (br s, 2H), 7.36 (s, 4H), 7.24 (m, 5H), 3.02 (m, 8H), and 1.93 (p, 2H, J = 7.2 Hz).

Anal. Calcd. for C₁₇H₂₀ CINS · HCl: C, 59.65; H, 6.19; N, 4.10. Found: C, 59.81; H, 6.06; N, 4.05.

As is apparent, various intermediate compounds may be prepared by appropriate modifications of the foregoing synthetic processes. These intermediates are accessible by other synthetic routes as well which would be familiar to one skilled in the art.

### Syntheses of Formula I Compounds

### A. Sulfides

### Example 9

### General Procedure for the Synthesis of Sulfides (Ia)

Sodium metal (2.3 g, 100 mmol) was added dropwise under nitrogen to 100 mL of absolute ethanol. After all of the sodium metal had been consumed, 90 mmcol of the appropriate thiophenol 1 was added and the resulting solution and stirred 5 min at room temperature. At this time the appropriate halide, (100 mmol, 4-bromo-1-chlorobutane, 5-bromo-1-chloropentane, 6-bromo-1-chlorohexane, 3-bromo-1-chloropropane, or 1,2-dibromoethane) was added and the solution allowed to stir overnight at room temperature. The ethanol was removed in vacuo and the residue taken up in methylene chloride and washed with water and brine. After drying over anhydrous magnesium sulfate and filtration, the filtrate was concentrated in vacuo to generally give the haloalkyl sulfide V as a clear liquid. These were used without further purification.

The haloalkyl sulfide V was taken up in 150 mL of acetonitrile containing 13.8 g (100 mmol) of micropulverized anhydrous potassium carbamate and approximately 50 mg of sodium iodide. The appropriate amine VI (125 mmol) was added and the resulting suspension allowed to reflux under nitrogen 24 h. After the reaction was judged complete by tlc analysis, it was poured into 500 mL of H₂O and extracted with CH₂Cl₂. The combined organic extracts were washed with H₂O and brine, and then dried over anhydrous magnesium sulfate. Filtration and concentration in vacuo gave the crude sulfides which were purified by flash chromatography (silicia gel, ethyl acetate/hexane solutions). The purified free base sulfides Ia were converted into their salt forms by treatment with either hydrochloric acid, maleic acid, fumaric acid, or oxalic acid and after recrystallization from acetonitrile/ether isolated as crystalline solids.

The following sulfides (Ia) were prepared using these procedures.

### Example 10

### N-Methyl-N-[3-(Phenylthio)propyl]benzeneethanamine maleate

Isolated as a colorless crystalline salt: mp 77.5-79°C; ¹H NMR (DMSO-d₆) δ 7.25 (m, 10H), 6.01 (s, 2H), 3.21 (m, 4H), 3.01 (t, 2H, J = 7.1 Hz), 2.90 (m, 2H), 2.78 (s, 3H), and 1.92 (m, 2H).

Anal. Calcd. for C₁₈H₂₃NS·C₄H₄O₄: C, 65.81; H, 6.78; N, 3.49. Found: C, 65.97; H, 6.78; N, 3.45.

### Example 11

### N-[4-(Phenylthio)butyl]-N-methylbenzeneethanamine hydrochloride

Obtained as a white solid after recrystallization from isopropanol/ether: mp 83-84°C; ¹H NMR (CD₃OD) δ 1.69 (m, 2H), 2.89 (s, 3H), 2.99 (t, 2H, J = 7 Hz), 3.06 (m, 2H), 3.16 (m, 2H), 7.18 (m, 1H), 7.24-7.37 (m, 9H).

Anal. Calcd. for C₁₉H₂₅NS·HCl: C, 67.93; H, 7.80; N, 4.17. Found: C, 67.63; H, 7.93; N, 4.24.

### Example 12

### N-[3-[(4-Fluorophenyl)thio]propyl]-N-methyl-benzeneethanamine hydrochloride

Isolated as a colorless silky white solid after recrystallization from acetonitrile/ether: mp 95-96°C; ¹H NMR (DMSO-d₆) δ 11.13 (br s, 1H), 7.43 (m, 2H), 7.24 (m, 7H), 3.22 (m, 4H), 3.01 (m, 4H), 2.72 (s, 3H), and 1.96 (m, 2H).

Anal. Calcd. for C₁₈H₂₂FNS·HCl: C, 63.61; H, 6.83; N, 4.13. Found: C, 63.70; H, 6.91; N, 4.13.

### Example 13

### N-[3-[4-Aminophenyl)thio]propyl]-N-methylbenzeneethanamine oxalate

Isolated as a creamy white solid after recrystallization from aqueous ethanol: mp 115-116°C (sintered); ¹H NMR (DMSO-d₆) δ 7.25 (m, 5H), 7.12 (d, 2H, J= 8.3 Hz), 6.51 (d, 2H, J = 8.5 Hz), 3.18 (m, 4H), 2.91 (m, 2H), 2.75 (s, 3H), 2.72 (m, 2H), and 1.81 (m, 2H).

Anal. Calcd. for C₁₈H₂₄N₂S·1.65 C₂H₂O₄: C, 56.98; H, 6.13; N, 6.24. Found: C, 56.94; H, 6.16; N, 6.29.

### Example 14

### N-[4-[(2-Aminophenyl)thio]butyl]-N-methylbenzenethanamine oxalate

Obtained as a white crystalline solid after recrystallization from isopropyl/water: mp 192-195°C; ¹H NMR (DMSO-d₆) δ 10.59 (s, 1H); 8.25 (d, 1H, J = 7.9 Hz), 7.60 (d, 1H, J = 6.6 Hz), 7.31 (m, 6H), 7.12 (t, 1H, J = 6.6 Hz), 3.22 (m, 2H), 3.05 (m, 4H), 2.87 (t, 2H, J = 6.0 Hz), 2.81 (s, 3H), 1.86 (m, 2H), and 1.48 (m, 2H).

Anal. Calcd. for C₁₉H₂₆N₂ S · 1.25 C₂H₂O₄: C, 60.47; H, 6.73; N, 6.56. Found: C, 60.85; H, 6.23; N, 6.37.

### Example 15

### N-[3-[(2-Aminophenyl)thio]propyl]-N-methylbenzeethanamine oxalate hydrate

Obtained as a white amorphous solid after recrystallization from isopropanol/water: mp 91-94°C, ¹H NMR (DMSO-d₆ ) δ 7.26 (m, 6H), 7.04 (t, 1H, J = 7.2 Hz), 6.74 (d, 1H, J = 7.5 Hz), 6.51 (t, 1H, J = 6.9 Hz), 3.14 (m, 4H), 2.92 (m, 2H), 2.76 (m, 2H), 2.71 (s, 3H), and 1.83 (m, 2H).

Anal. Calcd. for C₁₈H₂₄N₂S · C₂H₂O₄ · 0.1H₂O: C, 61.23; H, 6.73; N, 7.14; H₂O; 0.46. Found: C, 61.22; H, 6.67; N, 7.13; H₂O, 0.66.

### Example 16

### N-[4-[4-Aminophenyl)thio]butyl]-N-methylbenzeneethanamine oxalate

Obtained as a solid, m.p. 70-72°C.

Anal. Calcd. for C₁₉H₂₆N₂S · 1.4 C₂H₂O₄ · 0.1H₂O: C, 59.14; H, 6.60; N, 6.32; H₂O; 0.47. Found: C, 59.14; H, 6.47; N, 6.23, H₂O, 0.66.

The Ia compounds with R¹ = amino (Examples 13-16) serve as intermediates for synthesis of the Ia amide products (R¹ = R³ CONH).

### Example 17

### N-[2-[(4-Fluorophenyl)thio]ethyl]-N-methylbenzeneethanamine hydrochloride

Isolated as a colorless white solid after recrystallization from ether/acetonitrile: mp 113-114°C; ¹H NMR (DMSO-d₆) δ 11.47 (br s, 1H), 7.48 (m, 2H), 7.25 (m, 7H), 3.35 (m, 2H), 3.20 (m, 4H), 2.96 (m, 2H) and 2.71 (s, 3H);

Anal. Calcd. for C₁₇H₂₀FNS · HCl: C, 62.66; H, 6.50; N, 4.30. Found: C, 62.50; H, 6.25; N, 4.27.

### Example 18

### N-[4-[(4-Fluorophenyl)thio]butyl]-N-methylbenzeneethanamine hydrochloride

Isolated as a colorless white solid after recrystallization from acetonitrile/ether: mp 81-83°C; ¹H NMR (DMSO-d₆) δ 7.38 (m, 2H), 7.27 (m, 7H), 3.03 (m, 6H), 2.94 (t, 2H, J = 7.2 Hz), 2.73 (s, 3H), 1.80 (m, 2H) and 1.55 (m, 2H). Anal. Calcd. for C₁₉H₂₄FNS · HCl: C, 64.48; H, 7.13; N, 3.96. Found: C, 64.70; H, 7.29; N, 3.95.

### Example 19

### N-[5-[(4-Fluorophenyl)thio]pentyl]-N-methylbenzeneethanamine oxalate

Obtained as a colorless solid from the base (purified by column chromatography) in 96% yield: mp 124.5-125.5°C; ¹H NMR (DMSO-d₆) δ 7.60-7.21 (m, 7H), 7.18 (t, 2H, J = 8 Hz), 3.20-3.10 (m, 2H), 3.01-2.89 (m, 6H), 2.73 (s, 3H), 1.60-1.48 (m, 4H), 1.46-1.32 (m, 2H). .

Anal. Calcd. for C₂₀H₂₆FNS · C₂H₂O₄: C, 62.69; H, 6.69; N, 3.32. Found: C, 62.97; H, 6.88; N, 3.34.

### Example 20

### N-[6-[(4-Fluorophenyl)thio]hexyl]-N-methylbenzeneethanamine oxalate

Recrystallized from ethanol/ether, resulting in colorless crystals: mp 126.5-128°C; ¹H NMR (DMSO-d₆) δ 7.39-7.23 (m, 7H), 7.15 (t, 2H, J = 8 Hz), 3.20-3.10 (m, 2H), 3.01-2.80 (m, 6H), 2.73 (s, 3H), 1.60-1.47 (m, 4H), 1.46-1.56 (m, 4H).

Anal. Calcd. for C₂₁H₂₈FNS · C₂H₂O₄: C, 63.42; H, 6.94; N, 3.22. Found: C, 63.25; H, 6.88; N, 3.21.

### Example 21

### N-[3-[(4-Fluorophenyl)thio]propyl]-N-methylbenzenepropanamine

Purified by flash chromatography on silicia gel. The clean product, a pale yellow oil, had the following physical properties: ¹H NMR (CDCl₃) δ 7.36-6.93 (m, 9H), 2.92 (t, 2H, J = 11.6 Hz), 2.61 (t, J = 7.0 Hz, 2H), 2.34 (t, J = 7.6 Hz, 2H), 2.17 (s, 3H), 1.82-1.69 (m, 4H).

Anal. Calcd. for C₁₉H₂₄FNS: C, 71.88; H, 7.62; N, 4.41. Found: C, 71.70; H, 7.78; N, 4.39.

### Example 22

### N-Methyl-[3-[(1-naphthalenyl)thio]propyl]benzeneethanamine oxalate hydrate

Obtained after recrystallization from ethanol/water: mp 156-158°C; ¹H NMR (CDCl₃/DMSO-d₆) δ 8.18 (d, 1H, J = 8.0 Hz), 7.67 (d, 1H, J = 9.3 Hz), 7.50 (d, 1H, J = 8.1 Hz), 7.38 (m, 3H), 7.25 (m, 1H), 7.10 (m, 3H), 6.63 (d, 2H, J = 6.5 Hz), 2.85 (m, 8H), 2.54 (s, 3H), and 1.84 (m, 2H).

Anal. Calcd. for C₂₂H₂₅ SN · C₂H₂O₄ · 0.40 H₂O: C, 66.62; H, 6.48; N, 3.24; H₂O, 1.67. Found: C, 66.70; H, 6.40; N, 3.28; H₂O, 0.12.

### Example 23

### N-Methyl-N-[4-[(1-naphthalenyl)thio]butyl]benzeneethanamine oxalate

Obtained as a white solid after recrystallization from ethanol: mp 126-128°C; ¹H NMR (CDCl₃/DMSO-d₆) δ 7.98 (m, 1H), 7.46 (m, 1H), 7.37 (d, 1H, J = 8.0 Hz), 7.15 (m, 3H), 7.05 (m, 1H), 6.87 (m, 5H), 2.83 (m, 2H), 2.64 (m, 3H), 7.05 (m, 1H), 6.87 (m, 5H), 2.83 (m, 2H), 2.64 (m, 3H), 2.41 (s, 3H), 1.52 (m, 2H), and 1.30 (m, 2H). High resolution mass spectroscopy, calcd. m/e for C₂₃H₂₇ SN: 350.1942. Found: 350.1944.

### Example 24

### N-Methyl-N-[3-[(2-naphthalenyl)thio]propyl]benzeneethannamine maleate

Obtained as a white solid after recrystallization from acetonitrile: mp 101-104°C; ¹H NMR (CDCl₃) δ 7.75 (m, 4H), 7.47 (m, 3H), 7.24 (m, 3H), 7.05 (m, 2H), 3.29 (m, 2H), 3.19 (m, 2H), 3.07 (m, 2H), 2.97 (m, 2H), 2.76 (s, 3H), and 2.1 (m, 2H).

Anal. Calcd. for C₂₂H₂₅ SN · C₄H₄O₄: C, 69.15; H, 6.47; N, 3.10. Found: C, 69.03; H, 6.51; N, 3.11.

### Example 25

### N-Methyl-N-[4-[(2-naphthalenyl)thio]butyl]benzeneethanamine oxalate

Obtained as a white solid after recrystallization from ethanol: mp 158-159°C; ¹H NMR (DMSO-d₆) δ 7.84 (m, 4H), 7.47 (m, 3H), 7.29 (m, 5H), 3.09 (m, 4H), 3.03 (m, 2H), 2.72 (s, 3H), 1.79 (m, 2H) and 1.66 (m, 2H).

Anal. Calcd. for C₂₃H₂₇ NS · 1.5 C₂H₂O₄ : C, 63.54; H, 6.39; N, 2.96. Found: C, 63.30; H, 6.27; N, 2.89.

### Example 26

### N-[3-[(4-Chlorophenyl)thio]propyl]-N-methylbenzeneethanamine hydrochloride

A solution of the secondary amine product of Example 8 (17.8 g, 58.2 mmol) and 100 mL of ethyl formate was refluxed under N₂. After 5 h, tlc analysis indicated all of the amine had been converted into the formamide. The reaction was concentrated in vacuo to give 20.2 g of the crude formamide as a yellow oil.

This oil was taken up in 500 mL of THF and then treated under N₂ with 50 mL (0.10 mmol) of borane dimethyl sulfide complex (2.0 M in THF), and then stirred overnight at room temperature (15 h). The solution was quenched by careful addition of 50 mL of 10% aqueous hydrochloric acid (vol:vol). After refluxing 1 h, the solution was cooled to ambient and made basic with 10% aqueous sodium hydroxide (wt:vol). The phases were separated and the organic portion washed with H₂O and brine. After drying over anhydrous magnesium sulfate and filtration, the volatiles were removed in vacuo to give 17.3 g (54.1 mmol, 93% crude yield) of product as a pale yellow oil. A small portion of the free base was taken up in ether and converted to the hydrochloride salt by treatment with ethanolic hydrogen chloride. The salt was isolated as a colorless white solid after recrystallization from ethanol/ether: mp 106-107°C;
¹H NMR (DMSO-d₆) δ 11.21 (br s, 1H), 7.37 (s, 4H), 7.22 (m, 5H), 3.20 (m, 4H), 3.02 (m, 4H), 2.74 (s, 3H), and 2.00 (m, 2H).

Anal. Calcd. for C₁₈H₂₂ ClNS · HCl: C, 60.74; H, 6.51; N, 3.94. Found: C, 60.73; H, 6.47; N, 3.81.

### Example 27

### General Procedure for the Preparation of Amide-Substituted Ia Compounds

To a stirred, 0°C solution of an appropriate aminophenyl intermediate (e.g. Examples 13-16), and 10 mmol of triethylamine in 75 mL of methylene chloride was added 11 mmol of the requisite acylating agent (e.g. anhydride, acid chloride, chlorofomate, or isocyanate). The resulting solution was allowed to warm to room temperature and stir several hours or until tlc analysis indicated the reaction was complete. The reaction mixture was then poured into 200 mL of water. After separation, the organic portion was washed with H₂O, brine, and then dried over anhydrous magnesium sulfate. The solution was concentrated in vacuo and the resulting crude amides purified by flash chromatography (SiO₂: MeOH/EtOAc). These purified adducts were then converted to their hydrochloride or oxalate salts by treatment with the appropriate acid and recrystallized from ethanol/ether mixtures.

In this manner the following amide derivatives were prepared.

### Example 28

### N-[4-[[3-[N-Methyl-N-(2-phenylethyl)amino]propyl]thio] phenyl]acetamide hydrochloride hydrate

Obtained after crystallization from acetonitrile/ether: mp 135-147°C; ¹H NMR (DMSO-d₆) δ 10.88 (br s, 1H), 10.16 (s, 1H), 7.55 (m, 2H), 7.29 (m, 7H), 3.19 (m, 2H), 2.98 (m, 2H), 2.73 (s, 3H), 2.02 (s, 3H), and 1.93 (m, 2H).

Anal. Calcd. for C₂₀H₂₆N₂OS · HCl · 0.25 H₂O: C, 62.65; H, 7.23; N, 7.31; H₂O, 1.17. Found: C, 62.88; H, 7.18; N, 7.36; H₂ O, 1.30.

### Example 29

### N-[4-[4-[[N-Methyl-N-(2-phenylethyl)amino]butyl]thio] phenyl]acetamide oxalate hydrate

Obtained as a white solid after recrystallization from isopropanol/water: mp 134-136°C; ¹H NMR (DMSO-d₆) δ 7.54 (d, 2H, J = 8.6 Hz), 7.27 (m, 7H), 3.15 (m, 2H), 3.01 (m, 2H), 2.93 (m, 4H), 2.01 (s, 3H), 1.71 (m, 2H), and 1.55 (m, 2H).

Anal. Calcd. for C₂₁H₂₈N₂OS · C₂H₂O₄ · 0.25 H₂O: C, 61.24; H, 6.81; N, 6.21; H₂O, 1.00. Found: C, 60.30; H, 6.70; N, 6.07; H₂ O, 1.10.

### Example 30

### N-[2-[[3-N-Methyl-N-(2-phenylethyl)amino]propyl]thio] phenyl]acetamide oxalate hydrate

Obtained as a white solid after recrystallization from ethanol: mp 144-146°C; ¹H NMR (DMSO-d₆) δ 9.37 (br s, 1H), 7.30 (m, 2H), 7.21 (m, 5H), 3.10 (m, 4H), 2.91 (m, 4H), 2.69 (s, 3H), 2.05 (s, 3H), and 1.87 (m, 2H).

Anal. Calcd. for C₁₉H₂₆N₂OS ·1.5 C₂H₂O₄ · 0.18 H₂O: C, 56.36; H, 6.34; N, 5.77; H₂O, 0.38. Found: C, 56.66 H, 6.42; N, 6.27; H₂O, 0.71.

### Example 31

### N-[2-[[4-[N-Methyl-N-(2-phenylethyl)amino]butyl]thio] phenyl]acetamide oxalate hydrate

Obtained as a light brown solid after recrystallization from ethanol: mp 111-113°C; ¹H NMR (DMSO-d₆) δ 10.40 (br s, 1H), 9.30 (br s, 1H), 7.42 (m, 2H), 7.25 (m, 7H), 3.17 (m, 2H), 2.98 (m, 6H), 2.74 (s, 3H), 2.04 (s, 3H), 1.76 (m, 2H), and 1.53 (m, 2H).

Anal. Calcd. for C₂₁H₂₈N₂OS ·1.16 C₂H₂O₄ · 0.05 H₂O: C, 60.65; H, 6.64; N, 6.07; H₂O, 0.20. Found: C, 60.33 H, 6.60; N, 6.06; H₂ O, 0.20.

### Example 32

### N-[4-[3-[[N-Methyl-N-(2-phenylethyl)amino]propyl]thio] phenyl]benzamide oxalate

Obtained as a colorless solid after recrystallization from ethanol: mp 160-161°C; ¹H NMR (DMSO-d₆) δ 10.37 (s, 1H), 7.97 (d, 2H, J = 8.2 Hz), 7.81 (d, 2H, J = 8.5 Hz), 7.57 (m, 3H), 7.33 (m, 7H), 3.15 (m, 4H), 2.97 (m, 4H), 2.74 (s, 3H), and 1.94 (m, 2H).

Anal. Calcd. for C₂₅H₂₈N₂OS ·1.12 C₂H₂O₄ : C, 64.74; H, 6.04; N, 5.55. Found: C, 64.78 H, 5.70; N, 5.51.

### Example 33

### 2-Methyl-N-[4-[[3-[N-methyl-N-(2-phenylethyl)amino] propyl]thio]phenyl]propanamide oxalate hydrate

Obtained as a white crystalline solid after recrystallization from isopropanol and water: mp 193-195°C; ¹H NMR (DMSO-d₆) δ 9.94 (s, 1H), 7.58 (d, 2H, J = 8.7 Hz), 7.30 (m, 4H), 7.22 (m, 3H), 3.13 (m, 4H), 2.90 (m, 4H), 2.70 (s, 3H), 1.87 (m, 2H), and 1.06 (m, 6H).

Anal. Calcd. for C₂₁H₂₀N₂O₂S · C₂H₂O₄ · 0.01 H₂O: C, 51.53; H, 7.91; N, 6.24; H₂O, 0.59. Found: C, 61.24; H, 7.77; N, 6.21; H₂O, 0.22.

### Example 34

### N-[4-[[3-[N-Methyl-N-(2-phenylethyl)amino]propyl]thio] phenyl]propamide oxalate

Obtained as a white amorphous solid after recrystallization from isopropanol and water: mp 157-159°C; ¹H NMR (DMSO-d₆) δ 9.98 (s, 1H), 7.58 (d, 2H, J = 8.4 Hz), 7.27 (m, 7H), 3.12 (m, 4H), 2.92 (m, 4H), 2.69 (s, 3H), 2.28 (m, 2H), 1.87 (m, 2H), 1.05 (m, 3H).

Anal. Calcd. for C₂₁H₂₈N₂OS · C₂H₂O₄_{:} C, 61.86; H, 6.77; N, 6.27. Found: C, 61.84; H, 6.91; N, 6.22.

### Example 35

### N-[4-[[3-[N-Methyl-N-(2-phenylethyl)amino]propyl]thio] phenyl]heptanamide oxalate

Obtained as a white amorphous solid after recrystallization from isopropanol and water: mp 105-107°C; ¹H NMR (DMSO-d₆) δ 9.98 (s, 1H), 9.15 (br s, 1H), 7.59 (d, 2H, J = 8.7 Hz), 7.27 (m, 7H), 3.14 (m, 4H), 2.90 (m, 4H), 2.71 (s, 3H), 2.28 (t, 2H, J = 7.2 Hz), 1.89 (m, 2H), 1.53 (m, 2H), 1.24 (m, 6H), and 0.84 (m, 3H).

Anal. Calcd. for C₂₅H₃₇N₂OS · C₂H₂O₄_{:} C, 64.38; H, 7.81; N, 5.56. Found: C, 64.05; H, 7.74; N, 5.45.

### B. Sulfoxides

### Example 36

### General Procedure for the Synthesis of Sulfoxides (Ib)

To a stirred solution of 5.0 mmol of an appropriate Ia compound in 60 mL of methanol was added sodium periodate (1.5 g, 7.0 mmol) dissolved in 10 mL of water. The resulting white suspension was stirred at room temperature for 4 h and the poured into 300 mL of water. After extraction with methylene chloride, the combined organic portions were washed with brine and dried over anhydrous magnesium sulfate. The mixture was filtered and concentrated in vacuo to give an oil. Purification was accomplished by flash chromatography (SiO₂ : MeOH/EtOAc) to afford e.g. the pure sulfoxides of Examples 37 and 38 as shown below.

### Example 37

### N-Methyl-N-[3-(Phenylsulfinyl)propyl]benzeneethamine hydrate

Isolated as a pale yellow oil after column chromatography (SiO₂: chloroform/acetonitrile/ methanol): ¹H NMR (CDCl₃) δ 7.59-7.47 (m, 5H), 7.28-7.14 (m, 5H), 2.88-2.68 (m, 4H), 2.60-2.40 (m, 4H), 2.24 (s, 3H), 1.97-1.71 (m, 2H) .

Anal. Calcd. for C₁₈H₂₃NOS · 0.2 H₂O, C, 70.88; H, 7.74; N, 4.60. Found: C, 70.90; H, 7.52; N, 4.53.

### Example 38

### N-[3-[(4-Fluorophenyl)sulfinyl)propyl]-N-methylbenzeneethamine maleate hydrate

Obtained as a creamy white solid after trituration from ether: mp 92-94°C; ¹H NMR (DMSO-d₆) δ 7.73 (m, 2H), 7.46 (m, 2H), 7.25 (m, 5H), 6.03 (s, 2H), 3.16 (m, 6H), 2.85 (m, 2H), 2.78 (s, 3H), and 1.96 (m, 2H).

Anal. Calcd. for C₁₈H₂₂FNOS · C₄ H₄O₄ · 0.2 H₂O_{:} C, 60.18; H, 6.07; N, 3.20; H₂O, 0.82. Found: C, 60.11; H, 5.79; N, 3.21, H₂O, 0.85.

### C. Sulfones

### Example 39

### General Procedure for the Preparation of Sulfones (Ic)

To a solution of 10.0 mmol of an appropriate Ia sulfide salt dissolved in 75 mL of methanol, was added a solution of 11.0 mmol of OXONE® (KHSO₅ · KHSO₄ · K₂SO₄ /Aldrich Chemical Co) in 50 mL of water. The resulting suspension was stirred 3 h at room temperature and then poured into 400 mL of brine. After extraction with methylene chloride, the combined organic portions were washed with brine and dried over anhydrous magnesium sulfate, filtered and concentrated in vacuo to give the crude sulfone salt. The salts were either purified by recrystallization or treated with dilute sodium hydroxide to give the free base and then converted to the maleic, fumaric, or oxalic acid salt form.

The following sulfones were prepared using this route.

### Example 40

### N-Methyl-N-[3-(Phenylsulfonyl)propyl]benzeneethanamine hydrochloride

Isolated as a colorless crystalline material after recrystallization from ethanol/ether: mp 135.5-137°C; ¹H NMR (DMSO-d₆) δ 7.93-7.21 (m, 8H), 3.49-3.44 (m, 2H), 3.32-3.04 (m, 6H), 2.98 (t, 2H, J = 8.4 Hz), 2.75-2.74 (2s, total 3H), and 2.07-1.97 (m, 2H).

Anal. Calcd. for C₁₈H₂₃NO₂S · HCl: C, 61.09; H, 6.83; N, 3.96. Found: C, 60.91; H, 7.06; N, 3.93.

### Example 41

### N-[3-[4-(Fluorophenyl)sulfonyl]propyl]-N-methylbenzeneethanamine maleate

Obtained as a colorless white solid after recrystallization from acetonitrile: mp 137-138°C;
¹H NMR (DMSO-d₆) δ 7.96 (m, 2H), 7.50 (m, 2H), 7.29 (m, 5H), 6.01 (s, 2H), 3.40 (t, 2H, J = 7.5 Hz), 3.16 (m, 4H), 2.89 (m, 2H), 2.76 (s, 3H), and 1.93 (m, 2H).

Anal. Calcd. for C₁₈H₂₂NO₂S · C₄H₄O₄, C, 58.53; H, 5.81; N, 3.11. Found: C, 58.40; H, 5.67; N, 3.15.

### Example 42

### N-[2-[(4-Fluorophenyl)sulfonyl]ethyl]-N-methylbenzeneethanamine maleate

Obtained as a colorless white solid after recrystallization from acetonitrile: mp 152-153°C; ¹H NMR (DMSO-d₆) δ 8.01 (m, 2H), 7.53 (m, 2H), 7.33 (m, 2H), 7.26 (m, 3H), 6.08 (s, 2H), 3.84 (m, 2H), 3.29 (m, 2H) 3.14 (m, 2H), 2.85 (m, 2H), and 2.70 (s, 3H).

Anal. Calcd. for C₁₇H₂₀FNO₃S · C₄H₄O₄_{:} C, 57.66; H, 5.54; N, 3.21. Found: C, 57.82; H, 5.48; N, 3.20.

### Example 43

### N-[4-[(4-Fluorophenyl)sulfonyl]butyl]-N-methylbenzeneethanamine maleate

Isolated as a colorless solid after recrystallization from ether/ acetonitrile: mp 126-127°C; ¹H NMR (DMSO-d₆) δ 7.96 (m, 2H), 7.51 (m, 2H), 7.33 (m, 5H), 6.02 (s, 2H), 3.39 (m, 2H), 3.22 (m, 2H), 3.08 (m, 2H) 2.92 (m, 2H), 2.76 (s, 3H) 1.69 (m, 2H), and 1.58 (m, 2H).

Anal. Calcd. for C₁₉H₂₄FNO₂S · C₄H₄O₄_{:} C, 59.35; H, 6.07; N, 3.01. Found: C, 58.96; H, 5.96; N, 3.03.

### Example 44

### N-Methyl-N-[3-(1-naphthalenyl)sulfonyl]propyl]benzeneethanamine oxalate

Obtained as a white solid after recrystallization from ethanol/water: mp 175-176°C; ¹H NMR (CDCl₃) δ 8.63 (d, 1H, J = 8.4 Hz), 8.29 (d, 1H, J = 8.1 Hz), 8.18 (d, 1H, J = 8.1 Hz), 7.67 (m, 3H), 7.28 (m, 5H), 3.04 (m, 10H), and 2.30 (br s, 3H).

Anal. Calcd. for C₂₂H₂₅NO₂S · C₂H₂O₄: C, 62.01; H, 6.11; N, 3.14. Found: C, 62.31; H, 5.94; N, 2.99.

### Example 45

### N-Methyl-N-[3-(2-naphthalenyl)sulfonyl]propyl]benzeneethanamine fumarate hydrate

Obtained as a brown solid after recrystallization from acetonitrile: mp 143-145°C; ¹H NMR (CDCl₃) δ 8.48 (s, 1H), 7.92 (m, 5H), 7.69 (m, 2H), 7.20 (m, 7H), 3.22 (t, 2H, J = 7.22 Hz), 2.80 (m, 6H), 2.43 (s, 3H), 2.11 (m, 2H).

Anal. Calcd. for C₂₂H₂₅NO₂S · C₂H₂O₄ · 0.5 H₂O: C, 64.46; H, 6.06; N, 2.90; H₂O, 0.90. Found: C, 64.12; H, 6.10; N, 2.89; H₂O, 0.10.

### Example 46

### N-Methyl-N-[(4-(2-naphthalenyl)sulfonyl]butyl]benzeneethanamine fumarate hydrate

Obtained as a tan amorphous solid after recrystallization from acetonitrile: mp 204°C (dec); ¹H NMR (DMSO-d₆) δ 8.28 (s, 1H), 7.79 (m, 5H), 7.25 (s, 1H), 7.06 (m, 4H), 6.58 (s, 1), 2.94 (m, 10H), 2.35 (s, 3H), and 1.61 (m, 2H).

Anal. Calcd. for C₂₃H₂₇NO₂S · 2 C₂H₂O₄ · 1.3 H₂O: C, 58.39; H, 5.95; N, 2.19; H₂O, 3.75. Found: C, 58.32; H, 5.69; N, 2.25; H₂O, 3.30.

### Example 47

### N-[4-[(2-Naphthalenyl)sulfonyl]butyl]benzeneethanamine oxalate hydrate

Obtained as a brown amorphous solid after recrystallization from acetonitrile: mp 185-187°C; ¹H NMR (CDCl₃) δ 8.28 (s, 1H), 7.83 (m, 2H), 7.67 (m, 1H), 7.52 (m, 2H), 7.08 (m, 5H), 6.58 (s, 1H), 2.94 (m, 10H), and 1.61 (m, 2H).

Anal. Calcd. for C₂₂H₂₅NO₂S · 1.5 C₂H₂O₄ · 0.5 H₂O: C, 58.80; H, 5.53; N, 2.70; H₂O, 1.70. Found: C, 59.10; H, 5.60; N, 2.77; H₂O, 2.40.

### Example 48

### General Procedure for the Preparation of α-Substituted Sulfones (Ic; R² H)

To a cold, (-78°C), stirred, N₂-atmosphere solution of an appropriate Ic sulfone (10 mmol) dissolved in 50 mL of dry, O₂-free THF, was added via syringe, 4.4 mL (11 mmol) of n-butyl lithium (2.5 M in n-hexane, Aldrich Chemical Co.). The resulting yellow anion solution was stirred at -78°C for 15 min and then 11 mmol of an appropriate electrophile e.g. methyl iodide, ethyl bromide, etc. was added and stirred until the reaction was complete (as determined by tlc analysis). Saturated ammonium chloride solution was added to quench the reaction. Water and ether were then added and the layers separated. The organic portion was washed with water and brine, and then dried over anhydrous magnesium sulfate. The mixture was filtered and the filtrate concentrated in vacuo. The resulting oil was purified by flash chromatography (SiO₂:EtOAc/hexane) to give the purified products of Ic wherein R² is lower alkyl. These oils were converted to their crystalline salt forms as described hereinabove.

### Example 49

### N-[3-[(4-Fluorophenyl)sulfonyl]butyl]-N-methylbenzeneethanamine maleate

Isolated as a colorless white solid after recrystallization from acetonitrile: mp 114-119°C; ¹H NMR (DMSO-d₆) δ 7.90 (m, 2H), 7.49 (m, 2H), 7.29 (m, 5H), 5.98 (s, 2H), 3.40 (m, 1H), 3.17 (s, 4H), 2.86 (t, 2H, J = 8.5 Hz), 2.74 (s, 3H), 2.09 (m, 1H), 1.72 (m, 1H), and 1.09 (d, 3H, J = 6.8 Hz).

Anal. Calcd. for C₁₉H₂₄FNO₂S · C₄H₄O₄: C, 59.35; H, 6.07; N, 3.01. Found: C, 59.37; H, 5.96; N, 2.94.

### Biological Test Methods

Vascular smooth muscle preparations: Peripheral (rabbit and/or rat aorta) and central (rabbit basilar artery) in vitro examinations. Cylindrical segments were suspended under tension in an organ bath and contractions were measured isometrically, first in the presence of the desired agonist (cumulative dose response), then after wash out and re-equilibration, with the compound of interest added. The activity was expressed in terms of a K_{b} value.

Neuroreceptor binding profile: In vitro determination in rat brain homogenates. Aliquots of washed membranes were incubated with a low concentration of radioligand and increasing concentration of the compound of interest. The potency of binding were expressed as the concentration of the compound that will inhibit 50% of the specific binding. Schild analysis was performed in order to determine the competitive nature of binding to the polyamine site.

Centrally mediated serotonin (5-HT) efficacy:
Behavioral determination in the Mongolian gerbil. The functional activity of the compound of interest at central 5HT₁ₐ sites was investigated by means of the ability to induce the 5-HT syndrome. Activity is expressed as the dose that induces the syndrome in 50% of the animals.

Anoxic depolarization response: In vitro electrophysiological investigation in rat hippocampal slices. Hypoxic conditions were produced by switching the aeration of the medium bathing the slice from 95% O₂ and 5% CO₂ gas to 95% N₂ and 5% CO₂ which produces rapid depolarization in control slices. Concentrations of the compound of interest were evaluated in terms of ability to alter latency to anoxic depolarization (analyzed by ANOVA) and depolarization amplitude (Bonferroni-correct t-test).

Protection against anoxia in vivo: Rats are exposed to 100% nitrogen for one minute. The number of animals that are alive after two hours is recorded.

Global forebrain ischemia: Histopathology after a 15-minute bilateral carotid occlusion in the Mongolian gerbil. The survival period was 96 hours. Compound is administered (ip) at approximately 1, 24, 48, and 72 hours post-occlusion. Fixed coronal sections at the level of the dorsal hippocampus were stained for Nissl substance and the hippocampal formation is analyzed for the amount of cell loss using a 5 point rating scale. Differences in group mean damage scores were analyzed using the Mann Whitney U test.

Focal ischemia: Measurement of infarct volume after permanent occlusion of the middle cerebral artery in the spontaneously hypertensive rat. The middle cerebral artery was electrocoagulated at its proximal end. Twenty-four hours later the brains were perfused and incubated with a vital dye. Cortical infarct is quantified from thick coronal sections using computer assisted planimetry. Group mean values are compared using the Student's t-test.

## Claims

1. A compound of Formula I and its pharmaceutically acceptable acid addition salts thereof 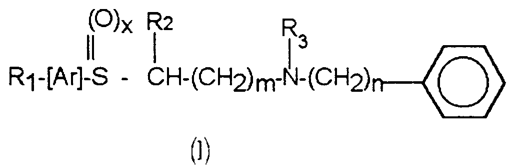 wherein Ar is naphthyl or phenyl;
R¹ is hydrogen, fluoro or R⁴CONH-;
R² is hydrogen or C₁₋₆ alkyl;
R³ is C₁₋₆ alkyl;
R⁴ is C₁₋₆ alkyl or phenyl- C₁₋₆ alkyl;
x is zero or the integers 1 and 2;
m is selected from the integers 1 to 6; and
n is selected from the integers 2 and 3.

2. A compound of claim 1 wherein x is zero.

3. A compound of claim 2 selected from the group consisting of N-methyl-N-[3-(phenylthio)propyl] benzeneethanamine; N-[4-(phenylthio)butyl]-N-methylbenzeneethanamine; N-[3-[(4- fluorophenyl)thio]propyl]-N-methyl-benzeneethanamine; N-[2-[(4- fluorophenyl)thio]ethyl]-N-methylbenzeneethanamine; N-[4-[(4- fluorophenyl)thio]butyl]-N-methylbenzeneethanamine; N-[5-[(4-fluorophenyl)thio]-pentyl]-N-methylbenzeneethanamine; N-[6-[(4-fluorophenyl)thio]hexyl]-N-methylbenzeneethanamine; N-[3-[(4-fluorophenyl)thio]propyl]-N-methylbenzenepropanamine; N-methyl-[3-[(1-naphthalenyl)thio] propyl]benzeneethanamine; N-methyl-N-[4-[(1-naphthalenyl)thio]butyl]benzeneethanamine; N-methyl-N-[3-[(2-naphthalenyl)thio]propyl] benzeneethanamine; N-methyl-N-[4-[(2-naphthalenyl)thio]butyl]benzeneethanamine; N-[4-[[3-[N-methyl-N-(2-phenylethyl)amino]propyl] thio]phenyl]acetamide; N-[4-[4-[[N-methyl-N-(2-phenylethyl)amino]butyl]thio]phenyl] acetamide; N-[2-[[3-N-methyl-N-(2-phenylethyl) amino]propyl]thio]phenyl]acetamide; N-[2-[[4-[N-methyl-N-(2-phenylethyl)amino]butyl]thio]phenyl] acetamide; N-[4-[3-[[N-methyl-N-(2-phenylethyl) amino]propyl]thio]phenyl]benzamide; 2-methyl-N-[4-[[3-[N-methyl-N-(2-phenylethyl)amino] propyl]thio]phenyl]propanamide; N-[4-[[3-[N-methyl-N-(2-phenylethyl)amino]propyl]thio]phenyl]propamide; N-[4-[[3-[N-methyl-N-(2-phenylethyl)amino]propyl] thio]phenyl]heptanamide.

4. A compound of claim 1 wherein x is 2.

5. A compound of claim 4 selected from the group consisting of N-methyl-N-[3-(phenylsulfonyl) propyl]benzeneethanamine; N-[3-[4-(fluorophenyl)sulfonyl]propyl]-N-methylbenzeneethanamine; N-[2-[(4-fluorophenyl)sulfonyl]ethyl]-N-methylbenzeneethanamine; N-[4-[(4-fluorophenyl) sulfonyl]butyl]-N-methylbenzeneethanamine; N-methyl-N-[3-(1-naphthalenyl)sulfonyl]propyl] benzeneethanamine; N-methyl-N-[3-(2-naphthalenyl) sulfonyl]propyl]benzeneethanamine; N-methyl-N-[(4-(2-naphthalenyl)sulfonyl]butyl]benzeneethanamine; N-[4-[(2-naphthalenyl)sulfonyl]butyl] benzeneethanamine; N-[3-[(4-fluorophenyl)sulfonyl] butyl]-N-methylbenzeneethanamine.

6. The compound of claim 3, N-[3-[(4- fluorophenyl)thio]propyl]-N-methyl-benzeneethanamine.

7. A pharmaceutical formulation which comprises as an active ingredient a compound of Formula I, as claimed in any one of Claims 1 to 6, associated with one or more pharmaceutically acceptable carriers, excipients or diluents therefor.

8. The pharmaceutical formulation of claim 7 wherein the compound of Formula I is N-[3-[(4- fluorophenyl)thio]propyl]-N-methyl-benzeneethanamine.

9. A compound of Formula I, as claimed in any one of claims 1 to 6, for use in protecting CNS tissue from ischemia.

10. The compound of Formula I which is N-[3-[(4-fluorophenyl)thio]propyl]-N-methyl-benzeneethanamine for use in protecting CNS tissue from ischemia.
